# EUROPEAN PATENT SPECIFICATION

(11) **EP 2 653 186 B1**
(45) Date of publication and mention of the grant of the patent: **12.08.2020**
(21) Application number: 11847959.1
(22) Date of filing: 16.12.2011
(51) Int. Cl.: A61M 37/00, B81C 1/00, A61K 9/00

(54) **METHOD FOR MANUFACTURING MICROSTRUCTURE BODY**
VERFAHREN ZUR HERSTELLUNG EINES MIKROSTRUKTURKÖRPERS
PROCÉDÉ DE FABRICATION D'UN CORPS À MICROSTRUCTURE

(30) Priority: 17.12.2010 KR 20100130169
(43) Date of publication of application: 23.10.2013
(73) Proprietor: Raphas Co., Ltd., Seoul 121-912 (KR)
(72) Inventor: JUNG, Hyungil, Seoul 122-080 (KR); JEONG, Do Hyeon, Seoul 121-901 (KR); KIM, Jung Dong, Seoul 152-828 (KR); KIM, Miroo, Seoul 152-100 (KR)
(74) Representative: Grünecker Patent- und Rechtsanwälte PartG mbB
(86) International application number: PCT/KR2011/009722
(87) International publication number: WO 2012/081933

(56) References cited:
- WO-A1-2006/018642
- WO-A1-2010/137319
- KR-A- 20100 038 071
- KR-B1- 100 781 702
- KR-B1- 100 793 615
- US-A1- 2009 053 472
- US-B1- 6 334 856

## Description

### [Technical Field]

The present invention relates to a method of manufacturing a microstructure such as a microneedle using a wind blowing technique.

### [Background Art]

While various drugs and phytonutrients for treating diseases have been developed, in delivery into a human body, they still have aspects in need of improvement such as transmitting across biological barriers (e. g., skin, oral mucosa, blood-brain barriers, etc.) and efficiency of drug delivery.

Generally, drugs and phytonutrients are orally administered in tablet or capsule formulation. However, since various drugs are digested or absorbed in the gastrointestinal tract or lost due to mechanisms occurring in the liver, these drugs cannot be effectively delivered through only such oral administration. In addition, some drugs cannot be effectively diffused through an intestinal mucosa. Furthermore, drug compliance in patients becomes a problem (for example, in patients who need to take drugs at predetermined intervals, or critical patients who cannot take drugs).

Another common technique for delivering drugs and phytonutrients is using a conventional needle. This technique is more effective than oral administration, but may cause pain in an injected area, local damage to skin, bleeding, and infection in the injected area.

To solve these problems, various microstructures including a microneedle have been developed. The microneedle currently developed has been used to deliver drugs to a human body, collect blood, and detect analytes from the human body.

The microneedle is characterized by piercing the skin without pain and having no external injury as compared with a conventional needle. For the painless piercing of the skin, it is important to determine the diameter of a top of the microneedle for the needle's minimum sharpness. In addition, since the microneedle needs to pierce 10 to 20-µm of the stratum corneum, which is the most potent barrier of skin, the microneedle needs to have sufficient physical hardness. Furthermore, a reasonable length of the microneedle to deliver drugs to capillaries should also be considered so as to increase the efficiency of drug delivery.

Conventionally, since an in-plane type microneedle ("Silicon-processed Microneedles," Journal of Microelectrochemical Systems 8, 1999) has been suggested, various types of microneedles have been developed. According to the method of manufacturing an out-of-plane type solid microneedle using an etching technique (disclosed in U. S. Patent Publication No. 2002138049, entitled "Microneedle Devices and Methods of Manufacture and Use Thereof'), a solid silicon microneedle was manufactured to have a diameter of 50 to 100 µm and a length of 500 µm. However, the microneedle could not pierce skin without pain, and had difficulty in delivery of drugs and cosmetic ingredients to a target region.

Meanwhile, Prausnitz (Georgia University, U.S.A.) suggested a method of manufacturing a biodegradable polymer microneedle by producing a template by performing etching or photolithography on glass (Biodegradable Polymer Microneedles: Fabrication, Mechanics and Transdermal Drug Delivery, Journal of Controlled Release 104, 2005, 5166). Further, in 2006, a method of manufacturing a biodegradable solid microneedle by loading a material manufactured in a capsule type at an end of a template manufactured by photolithography was suggested (Polymer Microneedles for Controlled-Release Drug Delivery, Pharmaceutical Research 23, 2006, 1008). According to the above-mentioned method, a drug which can be manufactured in a capsule type can be easily loaded, but when a large amount of such a drug is loaded, the microneedle is degraded in hardness, and thus there is a limit to the application to a drug that needs to be administered in a large dose.

In 2005, an absorbable microneedle was manufactured by Nano Device and Systems Inc. (Japanese Patent Publication No. 2005154321; and "Sugar Micro Needles as Transdermic Drug Delivery System," Biomedical Microdevices 7, 2005, 185). Such an absorbable microneedle is used in drug delivery or cosmetics without removal of the microneedle inserted intradermally. According to the above-mentioned method, a composition prepared by mixing maltose and a drug was applied to a template and then solidified so as to manufacture a microneedle. The Japanese Patent discloses that an absorbable microneedle for transdermal absorption of drugs is manufactured. However, the transdermal delivery of the drugs was accompanied by pain. In addition, due to a technical limit in the manufacture of a template, it was impossible to manufacture a microneedle whose top has a suitable diameter to achieve the painless absorption and which has a length required for effective drug delivery, that is, a length of 1 mm or more.

A biodegradable microneedle suggested by Prausnitz (Georgia University, U.S.A.) in 2008 was manufactured using a polydimethylsiloxane (PDMS) template and a material prepared by mixing polyvinylpyrrolidone (PVP) and methacrylic acid (MAA) (Minimally Invasive Protein Delivery with Rapidly Dissolving Polymer Microneedles, Advanced Materials 2008, 1). Further, a microneedle was manufactured by injecting carboxymethylcellulose into a pyramid-structure template (Dissolving Microneedles for Transdermal Drug Delivery, Biomaterials 2007, 1). However, the method using a template has a limit in that a new template and mold need to be manufactured in a complicated process to adjust a diameter and length of a microneedle. In addition, the process of manufacturing a microneedle by injecting a material into a template is complicated and takes a long time.

In 2008, in U.S. Patent No. 2008015742A1 issued to Mukai et al., Japan, an apparatus and method of manufacturing a skin needle using a pin structure was disclosed. This method includes melting a viscous material on a base of a substrate, and pulling the viscous material using tensile force by means of a pin. Since the method involves pulling a material which is melted by heat or is viscous using a pin structure, it further requires a process of manufacturing a new pin structure according to a desired pattern, resulting in an increased production cost, and, the heating process makes it difficult to load various biomedicines (hormones, vaccines, other protein medicines, etc.) which are sensitive to heat.

Meanwhile, skin is composed of a stratum corneum (<20 µm), an epidermis (<100 µm), and a dermis (300 to 2,500 µm), which are sequentially stacked from the outer layer of the skin. Accordingly, to deliver drugs and phytonutrients to a specific layer of the skin without pain, the microneedle needs to be manufactured to have a top diameter of approximately 30 µm, an effective length of 200 to 2,000 µm, and a sufficient hardness to pierce skin, which is also effective in delivery of drugs and cosmetic ingredients. In addition, to deliver drugs and phytonutrients by a biodegradable solid microneedle, it is necessary to exclude processes of destroying activities of the drugs and phytonutrients such as high heat treatment, treatment with an organic solvent, etc. from the process of manufacturing a microneedle.

Conventional solid microneedles are manufactured using limited materials such as silicon, polymers, metal, glass, etc. due to the limit of the manufacturing method. Use of the manufacturing method using a molding technique causes a complicated and time-consuming process, which has disadvantages of the degeneration of drugs, insufficient hardness of the microneedles and the loss of drugs. Accordingly, there are ongoing demands for a method of manufacturing a microneedle which has a thin diameter to pierce skin without pain and a sufficient length to deeply penetrate into skin, has a sufficient hardness without a particular limit to a material, and can minimize the loss of drugs.

A method for producing a microneedle made of resin, which may be regarded as a closest prior art, is disclosed in WO 2006/018642 A1. In order to do so a molten resin is discharged to a base plate one or more times, and the attached molten resin is stretched to form a minute needle. A further method for producing microneedles is disclosed in US 2009/053472 A1. In order to do so a stamp face is contacted with a patterned face, wherein the patterned face having a flowable substrate thereon. While the stamp face is separated from the patterned face, the microneedles are generated. A further method for producing a microneedle is disclosed in WO 2006/018642 A1. In order to do so a substance is deposited on a first substrate. From this substrate, a needle is produced.

### [Disclosure]

### [Technical Problem]

The present invention is directed to a method of manufacturing a microstructure which includes a functional material sensitive to heat, has sufficient hardness, and can minimize the loss of the functional material.

### [Technical Solution]

In particular, a method for manufacturing a microstructure is provided. This method has the features defined in claim 1. Further preferred embodiments are defined in the dependent claims.

### [Advantageous Effects]

According to the present invention, it is possible to manufacture microstructure including a functional material sensitive to heat, having sufficient hardness, and can minimize the loss of the functional material

### [Description of Drawings]

FIG. 1 illustrates a method of manufacturing a microstructure according to a first exemplary arrangement. The arrangement shown in Figure 1 is helpful for understanding the present invention;
FIG. 2 schematically illustrates a cross-section of a microneedle according to an elongation speed;
FIG. 3 illustrates spotting of different kinds of viscous compositions;
FIG. 4 illustrates a method of manufacturing a microstructure according to an arrangement of the present invention. The arrangement shown in figure 4 is an embodiment of the present invention; and
FIG. 5 illustrates a method of manufacturing a microstructure according to a third exemplary arrangement. The arrangement shown in figure 5 is helpful for the understanding of the present invention.

### [Mode for Invention]

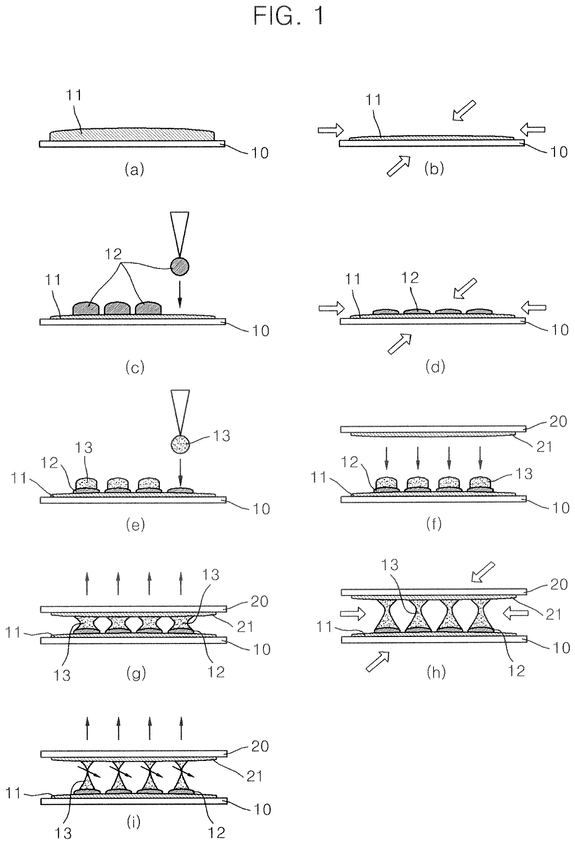

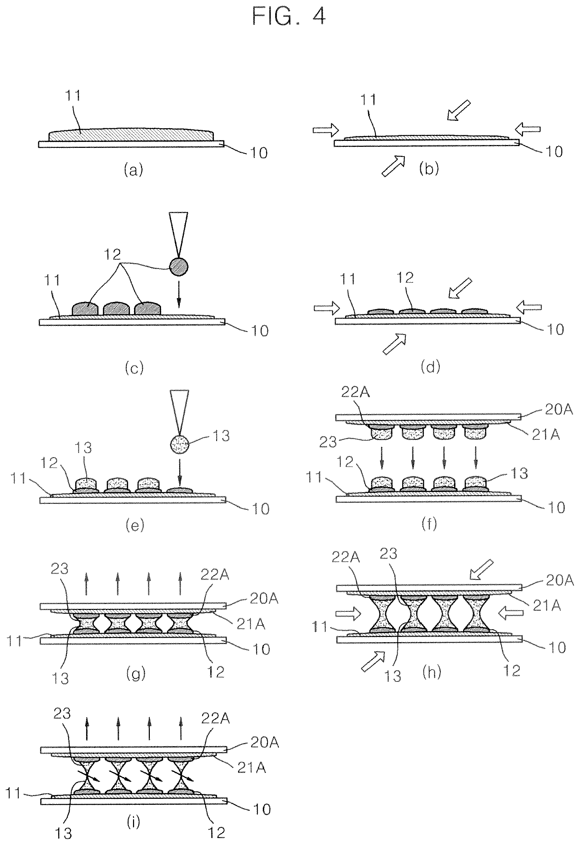

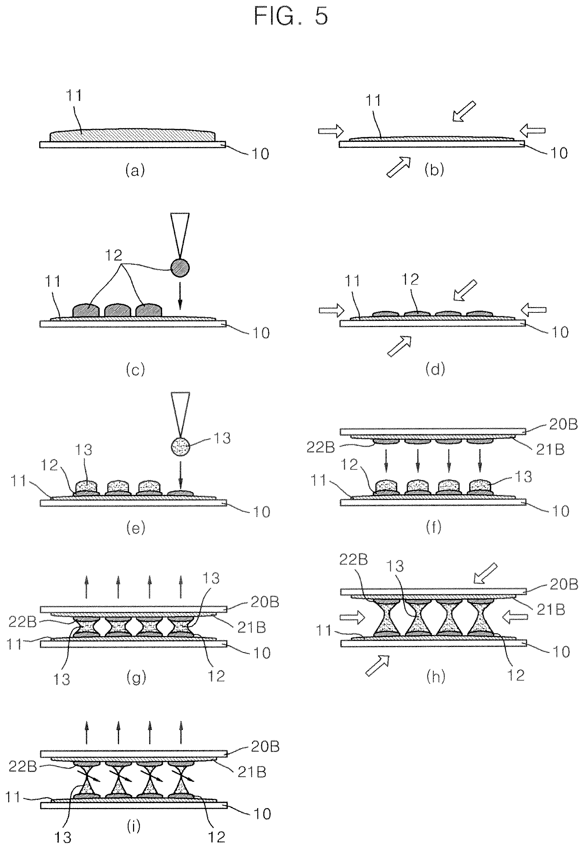

Exemplary arrangements of the present invention will be described in detail below with reference to the accompanying drawings. A method which is covered in claim 1 is shown in figure 4. The further arrangement shown in figure 1 and 5 are helpful for understanding the present invention.

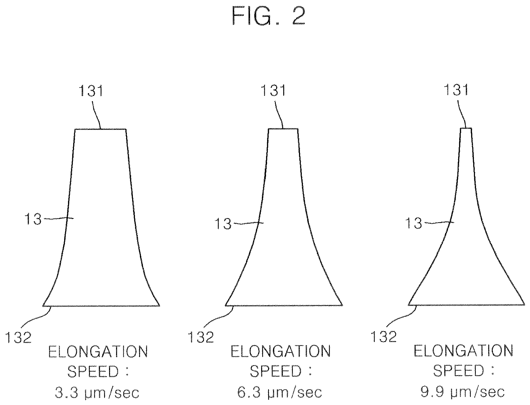

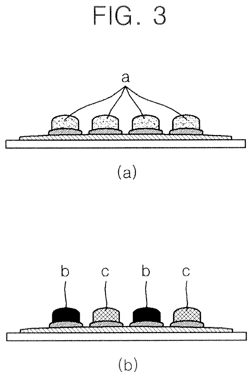

FIG. 1 illustrates a method of manufacturing a microstructure according to a first exemplary arrangement, FIG. 2 schematically illustrates a cross-section of a microneedle according to an elongation speed, and FIG. 3 illustrates spotting of different kinds of viscous compositions.

First, the present invention relates to a method of manufacturing a microstructure. Here, the microstructure includes a microneedle, a microblade, a microknife, a microfiber, a microspike, a microprobe, a microbarb, a microarray, and a microelectrode. Particularly, the method of manufacturing a microstructure according to the present invention is most suitable for manufacturing a microneedle, and a process of manufacturing a microneedle is illustrated below.

Referring to FIGS. 1 to 3, the method of manufacturing a microstructure includes formation of a bottom layer, formation of a base structure layer, spotting, contacting, elongation and solidification, and cutting.

In the formation of a bottom layer, as shown in FIGS. 1(a) and (b), a bottom layer is formed by coating a first viscous material 11 on a first substrate 10, and drying (solidifying) the material. Here, wind blows toward the substrate to facilitate solidification.

Here, the first substrate 10 is formed in a planar shape, and there is no particular limit to a material. For example, the substrate may be formed of a material such as a polymer, an organic material, a metal, a ceramic, a semiconductor, etc. However, when a medical-purpose microneedle is manufactured, the first substrate 10 may be formed of a material that is not harmful to the human body.

A first viscous material refers to a viscous material for forming various structures on a substrate, and second to fourth viscous materials also refer to viscous materials. While the first to fourth viscous materials are used to distinguish materials from each other throughout this specification, the materials for the first to fourth viscous materials are substantially the same as each other. The viscosity of the viscous materials may vary depending on the kind, concentration, and temperature of a material included in the composition, or the addition of a viscosity modifying agent, and may be suitably adjusted for the object of the present invention. Preferably, the viscosity of the viscous materials used in the present invention is 40,000 cSt or less.

In the manufacture of a microstructure used for a medical purpose such as a microneedle according to the exemplary arrangement, a viscous material may be a "biocompatible or biodegradable material." Here, the term "biocompatible material" refers to a material which is non-toxic and chemically inactive in the human body. The term "biodegradable material" refers to a material which can be degraded by a body fluid, enzyme or microorganism.

The viscous material according to the exemplary arrangement may exhibit viscosity when dissolved in a suitable solvent. That is, while, among visvous materials, some are viscous when melted by heat, a viscous material is preferably viscous when dissolved in a solvent to maximize an advantage of a non-heating process, which is one of the advantages to be described below.

The above-mentioned viscous materials include hyaluronic acid and a salt thereof, polyvinylpyrrolidone, polyvinylalcohol, cellulose polymer, dextran, gelatin, glycerin, polyethyleneglycol, polysorbate, propyleneglycol, povidone, carbomer, gum ghatti, guar gum, glucomannan, glucosamine, dammer resin, rennet casein, locust bean gum, microfibrillated cellulose, psyllium seed gum, xanthan gum, arabino galactan, gum arabic, alginic acid, gellan gum, carrageenan, karaya gum, curdlan, chitosan, chitin, tara gum, tamarind gum, tragacanth gum, furcelleran, pectin, pullulan, etc. Preferably, the viscous material used in the present invention is hydroxypropyl methylcellulose, hydroxyalkyl cellulose, ethyl hydroxyethyl cellulose, alkylcellulose, or carboxymethylcellulose, and more preferably, carboxymethylcellulose.

A solvent used to dissolve the above-mentioned viscous material may be, but is not particularly limited to, water, anhydrous or hydrous low alcohol having 1 to 4 carbon atoms, acetone, ethyl acetate, chloroform, 1,3-butyleneglycol, hexane, diethylether, or butylacetate, preferably water or low alcohol, and more preferably water.

In the formation of a base structure layer, as shown in FIGS. 1(c) and (d), a second viscous material 12 is spotted in spots spaced apart from each other on a bottom layer and solidified, thereby forming a base structure layer 12. Here, to facilitate solidification of the second viscous material 12, wind blowing may be performed. One of the above-mentioned kinds of the viscous materials may be used as the second viscous material, which may be the same as or different from the first viscous material.

Meanwhile, as an amount of the second viscous material used to form the base structure layer is increased, the diameter of the base structure layer is increased. As described below, to spot a large amount of a viscous composition including a functional material on the base structure layer, the base structure layer may have a larger diameter. This is because the base structure layer overflows with the viscous composition when the base structure layer has a small diameter and the large amount of the viscous composition is spotted, resulting in the loss of the functional material to be delivered to a living body. Meanwhile, after the base structure layer is formed by solidifying the second viscous material, a method of spotting the second viscous material again thereon and solidifying the viscous material is repeated to form the base structure layer to a desired height.

In the spotting, as shown in FIG. 1(e), for the base structure layer 12, a viscous composition 13 including a functional material is spotted. Here, the viscous composition 13 may be prepared by mixing the above-mentioned viscous material with the above-mentioned functional material. Particularly, for a microneedle as described in the exemplary arrangement, since the viscous composition 13 penetrates into skin, a viscous material having excellent biocompatibility and biodegradability such as carboxymethylcellulose may be used. Here, the functional material refers to a material serving a specific function such as a pharmacological action after penetrating into skin, and includes chemical drugs, protein medicines, peptide medicines, nucleic acid molecules for gene therapy, and nano-scale cosmetic ingredients (e.g., a wrinkle enhancer, a skin aging inhibitor, or skin whitener).

In the contacting, as shown in FIG. 1(f), the second substrate 20 is contacted with the adhesive composition 13. Here, the second substrate 20 is formed in a planar shape, and may be formed of the same material as the first substrate 10 as described above. Similar to the first substrate, a bottom layer 21 is formed on the second substrate 20 by solidifying a third viscous material, and the bottom layer 21 is contacted with the viscous composition 13. Meanwhile, the step of manufacturing the second substrate used in the contacting, that is, the step of forming the bottom layer 21 on the second substrate 20, may be performed previously for the sake of operation efficiency.

In the elongation and solidification, as shown in FIG. 1(g), the viscous composition 13 is elongated by moving the second substrate 20 with respect to the first substrate 10 (or moving the first substrate with respect to the second substrate) and then solidified as shown in FIG. 1(h). Here, the elongation and solidification may be performed by five methods.

The first method includes primarily elongating the viscous composition to a desired length (10 to 5000 µm) at a speed of 0.8 to 2000 µm/s while wind is not blown to the viscous composition, waiting for 1 to 100 seconds after the elongation stops, secondarily elongating the viscous composition to a desired length (10 to 5000 µm) at a speed of 0.8 to 2000 µm/s while wind is blown to the viscous composition at a wind speed of 1 to 100 m/s, and solidifying the viscous composition while wind is blown at a wind speed of 5 to 100 m/s. Here, the diameter of the microstructure is changed by the elongation speed, the wind speed, and the waiting time after the primary elongation. That is, as the waiting time becomes longer, diameters of the top and middle of the microstructure are increased.

The second method includes primarily elongating the viscous composition to a desired length (10 to 5000 µm) at a speed of 0.8 to 2000 µm/s while wind is not blown to the viscous composition, waiting for 1 to 100 seconds after the elongation stops, secondarily elongating the viscous composition to a desired length (10 to 5000 µm) at a speed of 0.8 to 2000 µm/s, and solidifying the viscous composition while wind is blown to the viscous composition at a wind speed of 5 to 100 m/s.

The third method includes elongating the viscous composition to a desired length (10 to 5000 µm) at a speed of 0.8 to 2000 µm/s while wind is blown to the viscous composition at a wind speed of 1 to 100 m/s, stopping the elongation and rapidly solidifying the viscous composition while wind is blown to the viscous composition at a wind speed of 5 to 100 m/s.

The fourth method includes elongating the viscous composition to a desired length (10 to 5000 µm) at a speed of 0.8 to 2000 µm/s while wind is not blown to the viscous composition, stopping the elongation and rapidly solidifying the viscous composition while wind is blown to the viscous composition at a wind speed of 5 to 100 m/s.

The fifth method includes primarily elongating the viscous composition to a desired length (10 to 5000 µm) at a speed of 0.8 to 2000 µm/s while wind is not blown to the viscous composition, waiting for 1 to 100 seconds after the elongation stops, blowing wind to the viscous composition at a wind speed of 1 to 100 m/s, secondarily elongating the viscous composition to a desired length (10 to 5000 µm) at a speed of 0.8 to 2000 µm/s while wind is blown continuously at a wind speed of 1 to 100 m/s, and solidifying the viscous composition while wind is blown at a wind speed of 5 to 100 m/s.

Here, as the waiting time after the primary elongation stops is increased, the middle diameter of the microstructure is increased, and the strength of the microstructure is relatively increased. Thus, an optimization operation is necessary to have sufficient strength for skin penetration by performing wind blowing by controlling the waiting time after the primary elongation. However, when an amount of the spotted viscous composition is 2 µl or less, the viscous material is solidified within a waiting time of 100 seconds or more. Therefore, most of the viscous material is broken before undergoing the secondary elongation. For this reason, it is difficult to form a microneedle having a suitable diameter and strength for skin penetration.

The above-described five elongation and solidification methods are suitably selected in consideration of characteristics such as the viscosity of the viscous composition and the shape of a microneedle to be formed.

In the cutting, as shown in FIG. 1(i), the second substrate 20 is rapidly moved with respect to the first substrate 10 to cut the viscous composition when the viscous composition is completely solidified (contrarily, the first substrate may be moved with respect to the second substrate). Meanwhile, the solidified viscous composition may be cut using a laser or a cutting apparatus.

Further, such a solidified and cut viscous composition 13, that is, the structural part 13, has a needle shape as shown in FIG. 2. However, the shape is slightly changed according to conditions for elongation (e.g., the elongation speed, the waiting time after the primary elongation, the wind speed, the time for wind blowing, etc.). Here, to effectively penetrate the structural part 13 into skin, a top diameter of the structural part 13 may be approximately 5 to 80 µm. The bottom of the structural part may have a diameter 10 to 1000 times larger than the top of the structural part, and the length from the bottom to the top of the structural part may be 200 to 3000 µm. Here, the top 131 of the structural part refers to a portion of the structural part 13 having the smallest diameter, and the bottom 12 of the structural part refers to a portion of the structural part having the largest diameter, that is, a portion of the structural part placed right on the base structure layer.

Meanwhile, as shown in FIG. 3(a), during the spotting of the viscous composition on the base structure layer, the same viscous composition a may be used, but as shown in FIG. 3(b), various kinds of viscous compositions b and c may be used. That is, by using viscous compositions including different functional materials, various kinds of drugs may be administered to skin at one time.

As described above, in the manufacturing method according to the exemplary arrangement, all of the processes are performed under non-heating conditions. Thus, a microneedle including heat-sensitive drugs such as protein drugs, peptide drugs, nucleic acid molecules for gene therapy, etc. can be manufactured.

Unlike the conventional method, the present invention does not need a process of newly manufacturing a pin structure according to a pattern or a process of washing a pin structure, and thus a production cost and production time can be reduced.

When a microneedle is formed directly on a substrate, a functional material loaded in a bottom part of the microneedle is not delivered to a living body and lost. However, according to the exemplary arrangement, the microneedle is formed on the base structure 12, which can minimize the loss of the functional material and aid in penetrating the microneedle 13 into skin.

According to the exemplary arrangement, by changing the kind of the functional material included in the viscous composition, microneedles including various kinds of functional materials may be manufactured on one first substrate.

FIG. 4 illustrates a method of manufacturing a microstructure according an embodiment of the present invention.

Referring to FIG. 4, a method of manufacturing a microstructure according to the exemplary arrangement is almost the same as described in the first exemplary arrangement. However, as shown in FIG. 4(f), according to the exemplary arrangement, a bottom layer 21A formed by solidifying a third viscous material and a base structure layer 22A formed by spotting a fourth viscous material on the bottom layer 21A and solidifying the material are formed on a second substrate 20A. Here, as the fourth viscous material, one of the above-mentioned viscous materials may be used. A viscous composition 23 including a functional material is spotted on the base structure layer. As shown in FIGS. 4(g) to (i), a viscous composition 13 spotted on a first substrate is adhered to the viscous composition 23 spotted on the second substrate, and then elongated, solidified, and cut. In the exemplary arrangement, since microneedles may be formed on two substrates (the first and second substrates) at one time, an operation speed is increased two fold.

Meanwhile, the second substrate 20A is substantially the same as the first substrate 10. The bottom layer 21A and the base structure layer 22A are formed on the second substrate, and a process of spotting the viscous composition 23 is the same as performed on the first substrate.

FIG. 5 illustrates a method of manufacturing a microstructure according to a third exemplary arrangement.

Referring to FIG. 5, a method of manufacturing a microstructure according to the exemplary arrangement is almost the same as described in the first exemplary arrangement. However, as shown in FIG. 5(f), a base structure layer 22B formed by solidifying a fourth viscous material in addition to a bottom layer 21B formed by solidifying a third viscous material may be formed on a second substrate 20B, and the base structure layer 22B may be adhered to a viscous composition 13, and then elongated.

According to the present invention, a microstructure which includes a functional material sensitive to heat, has sufficient hardness, and can minimize the loss of the functional material can be manufactured.

It will be apparent to those skilled in the art that various modifications can be made to the above-described exemplary arrangements. Thus, it is intended that the present invention covers all such modifications provided they come within the scope of the appended claims.

For example, a microstructure may be used to transmit or deliver an energy type such as thermal energy, light energy, electrical energy, etc. As one example, in photodynamic therapy, a microstructure can be used to induce light to a specific region in a human body to directly act on a tissue or act on a medium such as a light-sensitive molecule.

## Claims

1. A method of manufacturing a microstructure, comprising:
coating a first viscous material in a planar shape on a first planar substrate (10) and drying the material to form a bottom layer (11);
spotting a first viscous composition in a plurality of spots (13) spaced apart from each other on the bottom layer (11);
contacting a second planar substrate (20A) with the spotted first viscous composition;
relatively moving the second planar substrate (20) with respect to the first planar substrate (10), elongating the first viscous composition, and solidifying the elongated first viscous composition; and
cutting the solidified first viscous composition,
wherein the method further comprises the step of,
before spotting the first viscous composition on the bottom layer, spotting a second viscous material in a plurality of spots (12) spaced apart from each other on the bottom layer (11) of the first planar substrate (10) and solidifying the material to form a base structure layer (12),
wherein the first viscous composition is spotted on the base structure layer (12) of the first planar substrate (10),
**the method is characterized by**
preparing the second planar substrate (20A) including a bottom layer (21A) formed by coating and solidifying a third viscous material and a base structure layer (22A) formed by spotting a fourth viscous material in a plurality of spots spaced apart from each other on the bottom layer and then solidifying the material;
spotting a second viscous composition on the base structure layer (22A) of the second planar substrate (20A);
contacting the first viscous composition spotted on the base structure layer (12) of the first substrate (10) with the second viscous composition spotted on the base structure layer (22A) of the second substrate (21A);
relatively moving the second substrate (20A) with respect to the first substrate (10), elongating the first and second viscous composition, and solidifying the elongated first and second viscous composition; and cutting the solidified first and second viscous composition.

2. The method of claims 1, wherein the first and second viscous composition includes a functional material inducing a pharmacological effect in a human body.

3. The method of any one of claims 1 or 2, wherein elongating and solidifying the first and second viscous composition comprises:
primarily elongating the first and second viscous composition at a speed of 0.8 to 2000 µm/s while wind is not blown to the viscous composition;
waiting for 5 to 300 seconds after the elongation stops;
secondarily elongating the first and second viscous composition at a speed of 0.8 to 2000 µm/s while wind is blown to the viscous composition at a wind speed of 1 to 100 m/s; and solidifying the first and second viscous composition while wind is blown to the first and second viscous composition at a wind speed of 5 to 100 m/s.

4. The method of any one of claims 1 or 2, wherein elongating and solidifying the first and second viscous composition comprises:
primarily elongating the first and second viscous composition at a speed of 0.8 to 2000 µm/s while wind is not blown to the viscous composition;
waiting for 5 to 300 seconds after the elongation stops;
secondarily elongating the first and second viscous composition at a speed of 0.8 to 2000 µm/s while wind is not blown to the viscous composition; and
solidifying the first and second viscous composition while wind is blown to the first and second viscous composition at a wind speed of 5 to 100 m/s.

5. The method of any one of claims 1 or 2, wherein elongating and solidifying the first and second viscous composition comprises:
elongating the first and second viscous composition at a speed of 0.8 to 2000 µm/s while wind is blown to the viscous composition at a wind speed of 1 to 100 m/s; and
stopping the elongation and solidifying the first and second viscous composition while wind is blown to the viscous composition at a wind speed of 5 to 100 m/s.

6. The method of any one of claims 1 or 2, wherein elongating and solidifying the first and second viscous composition comprises:
elongating the first and second viscous composition at a speed of 0.8 to 2000 µm/s while wind is not blown to the first and second viscous composition; and stopping the elongation and solidifying the first and second viscous composition while wind is blown to the first and second viscous composition at a wind speed of 5 to 100 m/s.

7. The method of any one of claims 1-6, wherein, when the solidified first and second viscous composition is cut, a diameter of the top of the first and second viscous composition having the smallest diameter is 5 to 80 µm, and
a length from the bottom having the largest diameter to the top of the first and second viscous composition is 200 to 3000 µm.

## Patentansprüche

1. Verfahren zur Herstellung einer Mikrostruktur, umfassend:
Auftragen eines ersten viskosen Materials in einer planaren Form auf ein erstes planares Substrat (10) und Trocknen des Materials, um eine Bodenschicht (11) zu bilden;
Tupfen einer ersten viskosen Zusammensetzung in einer Vielzahl von voneinander beabstandeten Spots (13) auf die Bodenschicht (11);
Inkontaktbringen eines zweiten planaren Substrats (20A) mit der getupften ersten viskosen Zusammensetzung;
relatives Bewegen des zweiten planaren Substrats (20) in Bezug auf das erste planare Substrat (10), Dehnen der ersten viskosen Zusammensetzung und Verfestigen der getupften ersten viskosen Zusammensetzung; und
Schneiden der erstarrten ersten viskosen Zusammensetzung,
wobei das Verfahren ferner den folgenden Schritt umfasst,
vor dem Tupfen der ersten viskosen Zusammensetzung auf die Bodenschicht, Tupfen eines zweiten viskosen Materials in einer Vielzahl von voneinander beabstandeten Spots (12) auf der Bodenschicht (11) des ersten planaren Substrats (10) und Verfestigen des Materials zur Bildung einer Basisstrukturschicht (12),
wobei die erste viskose Zusammensetzung auf die Basisstrukturschicht (12) des ersten planaren Substrats (10) getupft ist,
das Verfahren ist **gekennzeichnet durch**
Vorbereiten des zweiten planaren Substrats (20A) mit einer Bodenschicht (21A), die durch Beschichten und Verfestigen eines dritten viskosen Materials gebildet wird, und einer Basisstrukturschicht (22A), die durch Tupfen eines vierten viskosen Materials in einer Vielzahl von voneinander beabstandeten Spots auf der Bodenschicht und anschließendes Verfestigen des Materials gebildet wird;
Tupfen einer zweiten viskosen Zusammensetzung auf die Basisstrukturschicht (22A) des zweiten planaren Substrats (20A);
Inkontaktbringen der ersten viskosen Zusammensetzung, die auf die Basisstrukturschicht (12) des ersten Substrats (10) getupft ist, mit der zweiten viskosen Zusammensetzung, die auf die Basisstrukturschicht (22A) des zweiten Substrats (21A) getupft ist;
relatives Bewegen des zweiten Substrats (20A) in Bezug auf das erste Substrat (10), Dehnen der ersten und zweiten viskosen Zusammensetzung und Verfestigen der gedehnten ersten und zweiten viskosen Zusammensetzung; und
Schneiden der erstarrten ersten und zweiten viskosen Zusammensetzung.

2. Verfahren nach Anspruch 1, wobei die erste und zweite viskose Zusammensetzung ein funktionelles Material enthält, das eine pharmakologische Wirkung in einem menschlichen Körper induziert.

3. Verfahren nach einem der Ansprüche 1 oder 2, wobei das Dehnen und Verfestigen der ersten und zweiten viskosen Zusammensetzung umfasst:
primäres Dehnen der ersten und zweiten viskosen Zusammensetzung bei einer Geschwindigkeit von 0,8 bis 2000 µm/s, während Wind nicht auf die viskose Zusammensetzung geblasen wird;
Warten von 5 bis 300 Sekunden, nachdem die Dehnung aufhört;
sekundäres Dehnen der ersten und zweiten viskosen Zusammensetzung bei einer Geschwindigkeit von 0,8 bis 2000 µm/s, während Wind mit einer Windgeschwindigkeit von 1 bis 100 m/s auf die viskose Zusammensetzung geblasen wird; und Verfestigen der ersten und zweiten viskosen Zusammensetzung, während Wind mit einer Windgeschwindigkeit von 5 bis 100 m/s auf die erste und zweite viskose Zusammensetzung geblasen wird.

4. Verfahren nach einem der Ansprüche 1 oder 2, wobei das Dehnen und Verfestigen der ersten und zweiten viskosen Zusammensetzung umfasst:
primäres Dehnen der ersten und zweiten viskosen Zusammensetzung bei einer Geschwindigkeit von 0,8 bis 2000 µm/s, während Wind nicht auf die viskose Zusammensetzung geblasen wird;
Warten von 5 bis 300 Sekunden, nachdem die Dehnung aufhört;
sekundäres Dehnen der ersten und zweiten viskosen Zusammensetzung bei einer Geschwindigkeit von 0,8 bis 2000 µm/s, während kein Wind auf die viskose Zusammensetzung geblasen wird; und
Verfestigung der ersten und zweiten viskosen Zusammensetzung, während Wind mit einer Windgeschwindigkeit von 5 bis 100 m/s auf die erste und zweite viskose Zusammensetzung geblasen wird.

5. Verfahren nach einem der Ansprüche 1 oder 2, wobei das Dehnen und Verfestigen der ersten und zweiten viskosen Zusammensetzung umfasst:
Dehnen der ersten und zweiten viskosen Zusammensetzung mit einer Geschwindigkeit von 0,8 bis 2000 µm/s, während Wind mit einer Windgeschwindigkeit von 1 bis 100 m/s auf die viskose Zusammensetzung geblasen wird; und
Stoppen der Dehnung und Verfestigen der ersten und zweiten viskosen Zusammensetzung, während Wind mit einer Windgeschwindigkeit von 5 bis 100 m/s auf die viskose Zusammensetzung geblasen wird.

6. Verfahren nach einem der Ansprüche 1 oder 2, wobei das Dehnen und Verfestigen der ersten und zweiten viskosen Zusammensetzung umfasst:
Dehnen der ersten und zweiten viskosen Zusammensetzung bei einer Geschwindigkeit von 0,8 bis 2000 µm/s, während Wind nicht auf die erste und zweite viskose Zusammensetzung geblasen wird; und Stoppen der Dehnung und Verfestigung der ersten und zweiten viskosen Zusammensetzung, während Wind mit einer Windgeschwindigkeit von 5 bis 100 m/s auf die erste und zweite viskose Zusammensetzung geblasen wird.

7. Verfahren nach einem der Ansprüche 1 bis 6, wobei, wenn die verfestigte erste und zweite viskose Zusammensetzung geschnitten wird, ein Durchmesser der Oberseite der ersten und zweiten viskosen Zusammensetzung mit dem kleinsten Durchmesser 5 bis 80 µm beträgt, und
eine Länge von der Unterseite mit dem größten Durchmesser bis zur Oberseite der ersten und zweiten viskosen Zusammensetzung 200 bis 3000 µm beträgt.

## Revendications

1. Procédé de fabrication d'une microstructure, comprenant :
le revêtement d'un premier matériau visqueux en une forme plane sur un premier substrat (10) plan et le séchage du matériau pour former une couche inférieure (11) ;
le dépôt en points d'une première composition visqueuse en une pluralité de points (13) espacés l'un de l'autre sur la couche inférieure (11) ;
la mise en contact d'un second substrat (20A) plan avec la première composition visqueuse en points ;
le déplacement de manière relative du second substrat (20) plan par rapport au premier substrat (10) plan, l'allongement de la première composition visqueuse, et la solidification de la première composition visqueuse allongée ; et
la découpe de la première composition visqueuse solidifiée,
où le procédé comprend en outre l'étape de,
avant le dépôt en points de la première composition visqueuse sur la couche inférieure, le dépôt en points d'un second matériau visqueux en une pluralité de points (12) espacés l'un de l'autre sur la couche inférieure (11) du premier substrat (10) plan et la solidification du matériau pour former une couche de structure de base (12),
la première composition visqueuse étant déposée en points sur la couche de structure de base (12) du premier substrat (10) plan,
**le procédé est caractérisé par**
la préparation du second substrat (20A) plan comprenant une couche inférieure (21A) formée par revêtement et solidification d'un troisième matériau visqueux et d'une couche de structure de base (22A) formée par dépôt en points d'un quatrième matériau visqueux en une pluralité de points espacés l'un de l'autre sur la couche inférieure et ensuite la solidification du matériau ;
le dépôt en points d'une seconde composition visqueuse sur la couche de structure de base (22A) du second substrat (20A) plan ;
la mise en contact de la première composition visqueuse déposée en points sur la couche de structure de base (12) du premier substrat (10) avec la seconde composition visqueuse déposée en points sur la couche de structure de base (22A) du second substrat (21A) ;
le déplacement de manière relative du second substrat (20A) par rapport au premier substrat (10), l'allongement de la première et de la seconde composition visqueuse, et la solidification de la première et de la seconde composition visqueuse allongées ; et
la découpe de la première et de la seconde composition visqueuse solidifiées.

2. Procédé selon la revendication 1, la première et la seconde composition visqueuse comprenant un matériau fonctionnel induisant un effet pharmacologique dans un corps humain.

3. Procédé selon l'une quelconque des revendications 1 ou 2, l'allongement et la solidification de la première et de la seconde composition visqueuse comprenant :
l'allongement de manière primaire de la première et de la seconde composition visqueuse à une vitesse de 0,8 à 2 000 µm/s lorsque du vent n'est pas soufflé sur la composition visqueuse ;
l'attente durant 5 à 300 secondes après que l'allongement s'arrête ;
l'allongement de manière secondaire de la première et de la seconde composition visqueuse à une vitesse de 0,8 à 2 000 µm/s lorsque du vent est soufflé au niveau de la composition visqueuse à une vitesse du vent de 1 à 100 m/s ; et
la solidification de la première et de la seconde composition visqueuse tandis que du vent est soufflé au niveau de la première et de la seconde composition visqueuse à une vitesse du vent de 5 à 100 m/s.

4. Procédé selon l'une quelconque des revendications 1 ou 2, dans lequel l'allongement et la solidification de la première et de la seconde composition visqueuse comprend :
l'allongement de manière primaire de la première et de la seconde composition visqueuse à une vitesse de 0,8 à 2 000 µm/s lorsqu'aucun vent n'est soufflé sur la composition visqueuse ;
l'attente durant 5 à 300 secondes après que l'allongement s'arrête ;
l'allongement de manière secondaire de la première et de la seconde composition visqueuse à une vitesse de 0,8 à 2 000 µm/s lorsqu'aucun vent n'est soufflé sur la composition visqueuse ; et
la solidification de la première et de la seconde composition visqueuse tandis que du vent est soufflé au niveau de la première et de la seconde composition visqueuse à une vitesse du vent de 5 à 100 m/s.

5. Procédé selon l'une quelconque des revendications 1 ou 2, dans lequel l'allongement et la solidification de la première et de la seconde composition visqueuse comprend :
l'allongement de la première et de la seconde composition visqueuse à une vitesse de 0,8 à 2 000 µm/s tandis que du vent est soufflé au niveau de la composition visqueuse à une vitesse du vent de 1 à 100 m/s ; et
l'arrêt de l'allongement et de la solidification de la première et de la seconde composition visqueuse tandis que du vent est soufflé au niveau de la composition visqueuse à une vitesse du vent de 5 à 100 m/s.

6. Procédé selon l'une quelconque des revendications 1 ou 2, dans lequel l'allongement et la solidification de la première et de la seconde composition visqueuse comprend :
l'allongement de la première et de la seconde composition visqueuse à une vitesse de 0,8 à 2 000 µm/s tandis que du vent n'est pas soufflé sur la première et la seconde composition visqueuse ; et l'arrêt de l'allongement et de la solidification de la première et de la seconde composition visqueuse tandis que du vent est soufflé au niveau de la première et de la seconde composition visqueuse à une vitesse du vent de 5 à 100 m/s.

7. Procédé selon l'une quelconque des revendications 1 à 6,
dans lequel, lorsque la première et la seconde composition visqueuse solidifiées sont coupées, un diamètre du sommet de la première et de la seconde composition visqueuse ayant le diamètre le plus petit est de 5 à 80 µm, et
une longueur depuis le fond ayant le diamètre le plus grand vers le sommet de la première et de la seconde composition visqueuse est de 200 à 3 000 µm.
